# EUROPEAN PATENT APPLICATION

(11) **EP 1 321 513 A1**
(43) Date of publication of application: **25.06.2003**
(21) Application number: 01130636.2
(22) Date of filing: 21.12.2001
(51) Int. Cl.: C11D 3/386, C11D 7/42

(54) **Subtilisin variants with improved characteristics**

(71) Applicant: Direvo Biotech AG, 50829 Köln (DE)
(72) Inventor: Kettling, Ulrich, Dr., c/o Direvo Biotech AG, 50829 Köln (DE); Koltermann, Andre, Dr., c/o Direvo Biotech AG, 50829 Köln (DE); Kensch, Oliver, Dr., c/o Direvo Biotech AG, 50829 Köln (DE); Kuhlemann, René, Dr., c/o Direvo Biotech AG, 50829 Köln (DE); Haupts, Ulrich, Dr., c/o Direvo Biotech AG, 50829 Köln (DE); Rarbach, Markus, Dr., c/o Direvo Bitoech AG, 50829 Köln (DE); Odenthal, Konrad, c/o Direvo Biotech AG, 50829 Köln (DE)
(74) Representative: Helbing, Jörg, Dr. Dipl.-Chem.

(57) **Abstract**

The present invention provides subtilisin variants with improved characteristics, including improved substrate affinity, catalytic activity, catalytic efficiency and stability under washing conditions as well as overall wash performance. The subtilisin variants are therefore useful additives in cleaning compositions.

SEQUENCE LISTING

<110> DIREVO Biotech AG

<120> Subtilisin Variants with Improved Characteristics

<130> 013221ep/JH/ml

<140>
<141>

<160> 9

<170> PatentIn Ver. 2.1

<210> 1
<211> 275
<212> PRT
<213> Bacillus amyloliquefaciens

<400> 1
Ala Gln Ser Val Pro Tyr Gly Val Ser Gln Ile Lys Ala Pro Ala Leu
  1               5                  10                  15

His Ser Gln Gly Tyr Thr Gly Ser Asn Val Lys Val Ala Val Ile Asp
             20                  25                  30

Ser Gly Ile Asp Ser Ser His Pro Asp Leu Lys Val Ala Gly Gly Ala
         35                  40                  45

Ser Met Val Pro Ser Glu Thr Asn Pro Phe Gln Asp Asn Asn Ser His
     50                  55                  60

Gly Thr His Val Ala Gly Thr Val Ala Ala Leu Asn Asn Ser Ile Gly
 65                  70                  75                  80

Val Leu Gly Val Ala Pro Ser Ala Ser Leu Tyr Ala Val Lys Val Leu
                 85                  90                  95

Gly Ala Asp Gly Ser Gly Gln Tyr Ser Trp Ile Ile Asn Gly Ile Glu
            100                 105                 110

Trp Ala Ile Ala Asn Asn Met Asp Val Ile Asn Met Ser Leu Gly Gly
        115                 120                 125

Pro Ser Gly Ser Ala Ala Leu Lys Ala Ala Val Asp Lys Ala Val Ala
    130                 135                 140

Ser Gly Val Val Val Val Ala Ala Ala Gly Asn Glu Gly Thr Ser Gly
145                 150                 155                 160

Ser Ser Ser Thr Val Gly Tyr Pro Gly Lys Tyr Pro Ser Val Ile Ala
                165                 170                 175

Val Gly Ala Val Asp Ser Ser Asn Gln Arg Ala Ser Phe Ser Ser Val
            180                 185                 190

Gly Pro Glu Leu Asp Val Met Ala Pro Gly Val Ser Ile Gln Ser Thr

195                 200                 205

Leu Pro Gly Asn Lys Tyr Gly Ala Tyr Asn Gly Thr Ser Met Ala Ser
    210                 215                 220

Pro His Val Ala Gly Ala Ala Ala Leu Ile Leu Ser Lys His Pro Asn
225                 230                 235                 240

Trp Thr Asn Thr Gln Val Arg Ser Ser Leu Gln Asn Thr Thr Thr Lys
                245                 250                 255

Leu Gly Asp Ser Phe Tyr Tyr Gly Lys Gly Leu Ile Asn Val Gln Ala
            260                 265                 270

Ala Ala Gln
        275

<210> 2
<211> 275
<212> PRT
<213> Bacillus subtilis

<400> 2
Ala Gln Ser Val Pro Tyr Gly Ile Ser Gln Ile Lys Ala Pro Ala Leu
1               5                   10                  15

His Ser Gln Gly Tyr Thr Gly Ser Asn Val Lys Val Ala Val Ile Asp
            20                  25                  30

Ser Gly Ile Asp Ser Ser His Pro Asp Leu Asn Val Arg Gly Gly Ala
        35                  40                  45

Ser Phe Val Pro Ser Glu Thr Asn Pro Tyr Gln Asp Gly Ser Ser His
    50                  55                  60

Gly Thr His Val Ala Gly Thr Ile Ala Ala Leu Asn Asn Ser Ile Gly
65                  70                  75                  80

Val Leu Gly Val Ser Pro Ser Ala Ser Leu Tyr Ala Val Lys Val Leu
                85                  90                  95

Asp Ser Thr Gly Ser Gly Gln Tyr Ser Trp Ile Ile Asn Gly Ile Glu
            100                 105                 110

Trp Ala Ile Ser Asn Asn Met Asp Val Ile Asn Met Ser Leu Gly Gly
        115                 120                 125

Pro Thr Gly Ser Thr Ala Leu Lys Thr Val Val Asp Lys Ala Val Ser
    130                 135                 140

Ser Gly Ile Val Val Ala Ala Ala Ala Gly Asn Glu Gly Ser Ser Gly
145                 150                 155                 160

Ser Thr Ser Thr Val Gly Tyr Pro Ala Lys Tyr Pro Ser Thr Ile Ala
                165                 170                 175

Val Gly Ala Val Asn Ser Ser Asn Gln Arg Ala Ser Phe Ser Ser Ala

180                 185                 190

Gly Ser Glu Leu Asp Val Met Ala Pro Gly Val Ser Ile Gln Ser Thr
        195                 200                 205

Leu Pro Gly Gly Thr Tyr Gly Ala Tyr Asn Gly Thr Ser Met Ala Thr
    210                 215                 220

Pro His Val Ala Gly Ala Ala Ala Leu Ile Leu Ser Lys His Pro Thr
225                 230                 235                 240

Trp Thr Asn Ala Gln Val Arg Asp Arg Leu Glu Ser Thr Ala Thr Tyr
                245                 250                 255

Leu Gly Asn Ser Phe Tyr Tyr Gly Lys Gly Leu Ile Asn Val Gln Ala
            260                 265                 270

Ala Ala Gln
        275


<210> 3
<211> 275
<212> PRT
<213> Bacillus subtilis

<400> 3
Ala Gln Ser Val Pro Tyr Gly Ile Ser Gln Ile Lys Ala Pro Ala Leu
  1               5                  10                  15

His Ser Gln Gly Tyr Thr Gly Ser Asn Val Lys Val Ala Val Ile Asp
             20                  25                  30

Ser Gly Ile Asp Ser Ser His Pro Asp Leu Asn Val Arg Gly Gly Ala
         35                  40                  45

Ser Phe Val Pro Ser Glu Thr Asn Pro Tyr Gln Asp Gly Ser Ser His
     50                  55                  60

Gly Thr His Val Ala Gly Thr Ile Ala Ala Leu Asn Asn Ser Ile Gly
 65                  70                  75                  80

Val Leu Gly Val Ser Pro Ser Ala Ser Leu Tyr Ala Val Lys Val Leu
                 85                  90                  95

Asp Ser Thr Gly Ser Gly Gln Tyr Ser Trp Ile Ile Asn Gly Ile Glu
            100                 105                 110

Trp Ala Ile Ser Asn Asn Met Asp Val Ile Asn Met Ser Leu Gly Gly
        115                 120                 125

Pro Thr Gly Ser Thr Ala Leu Lys Thr Val Val Asp Lys Ala Val Ser
    130                 135                 140

Ser Gly Ile Val Val Ala Ala Ala Ala Gly Asn Glu Gly Ser Ser Gly
145                 150                 155                 160

Ser Thr Ser Thr Val Gly Tyr Pro Ala Lys Tyr Pro Ser Thr Ile Ala

                165                 170                 175

Val Gly Ala Val Asn Ser Ser Asn Gln Arg Ala Ser Phe Ser Ser Ala
            180                 185                 190

Gly Ser Glu Leu Asp Val Met Ala Pro Gly Val Ser Ile Gln Ser Thr
        195                 200                 205

Leu Pro Gly Gly Thr Tyr Gly Ala Tyr Asn Gly Thr Ser Met Ala Thr
    210                 215                 220

Pro His Val Ala Gly Ala Ala Ala Leu Ile Leu Ser Lys His Pro Thr
225                 230                 235                 240

Trp Thr Asn Ala Gln Val Arg Asp Arg Leu Glu Ser Thr Ala Thr Tyr
                245                 250                 255

Leu Gly Asn Ser Phe Tyr Tyr Gly Lys Gly Leu Ile Asn Val Gln Ala
            260                 265                 270

Ala Ala Gln
        275

<210> 4
<211> 274
<212> PRT
<213> Bacillus licheniformis

<400> 4



<210> 5
<211> 269
<212> PRT
<213> Bacillus lentus

<400> 5
Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly

130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210> 6
<211> 275
<212> PRT
<213> Bacillus stearothermophilus

<400> 6
Ala Gln Ser Val Pro Tyr Gly Ile Ser Gln Ile Lys Ala Pro Ala Leu
  1               5                  10                  15

His Ser Gln Gly Tyr Thr Gly Ser Asn Val Lys Val Ala Val Ile Asp
             20                  25                  30

Ser Gly Ile Asp Ser Ser His Pro Asp Leu Asn Val Arg Gly Gly Ala
         35                  40                  45

Ser Phe Val Pro Ser Glu Thr Asn Pro Tyr Gln Asp Gly Ser Ser His
     50                  55                  60

Gly Thr His Val Ala Gly Thr Ile Ala Ala Leu Asn Asn Ser Ile Gly
 65                  70                  75                  80

Val Leu Gly Val Ser Pro Ser Ala Ser Leu Tyr Ala Val Lys Val Leu
                 85                  90                  95

Asp Ser Thr Gly Ser Gly Gln Tyr Ser Trp Ile Ile Asn Gly Ile Glu
            100                 105                 110

Trp Ala Ile Ser Asn Asn Met Asp Val Ile Asn Met Ser Leu Gly Gly
        115                 120                 125

Pro Ser Gly Ser Thr Ala Leu Lys Thr Val Val Asp Lys Ala Val Ser

130                 135                 140

Ser Gly Ile Val Val Ala Ala Ala Ala Gly Asn Glu Gly Ser Ser Gly
145                 150                 155                 160

Ser Ser Ser Thr Val Gly Tyr Pro Ala Lys Tyr Pro Ser Thr Ile Ala
                165                 170                 175

Val Gly Ala Val Asn Ser Ser Asn Gln Arg Ala Ser Phe Ser Ser Ala
            180                 185                 190

Gly Ser Glu Leu Asp Val Met Ala Pro Gly Val Ser Ile Gln Ser Thr
        195                 200                 205

Leu Pro Gly Gly Thr Tyr Gly Ala Tyr Asn Gly Thr Ser Met Ala Thr
    210                 215                 220

Pro His Val Ala Gly Ala Ala Ala Leu Ile Leu Ser Lys His Pro Thr
225                 230                 235                 240

Trp Thr Asn Ala Gln Val Arg Asp Arg Leu Glu Ser Thr Ala Thr Tyr
                245                 250                 255

Leu Gly Asn Ser Phe Tyr Tyr Gly Lys Gly Leu Ile Asn Val Gln Ala
            260                 265                 270

Ala Ala Gln
        275

<210> 7
<211> 268
<212> PRT
<213> Bacillus sp. YaB

<400> 7
Gln Thr Val Pro Trp Gly Ile Asn Arg Val Gln Ala Pro Ile Ala Gln
1               5                   10                  15

Ser Arg Gly Phe Thr Gly Thr Gly Val Arg Val Ala Val Leu Asp Thr
            20                  25                  30

Gly Ile Ser Asn His Ala Asp Leu Arg Ile Arg Gly Gly Ala Ser Phe
        35                  40                  45

Val Pro Gly Glu Pro Asn Ile Ser Asp Gly Asn Gly His Gly Thr Gln
    50                  55                  60

Val Ala Gly Thr Ile Ala Ala Leu Asn Asn Ser Ile Gly Val Leu Gly
65                  70                  75                  80

Val Ala Pro Asn Val Asp Leu Tyr Gly Val Lys Val Leu Gly Ala Ser
                85                  90                  95

Gly Ser Gly Ser Ile Ser Gly Ile Ala Gln Gly Leu Gln Trp Ala Ala
            100                 105                 110

Asn Asn Gly Met His Ile Ala Asn Met Ser Leu Gly Ser Ser Ala Gly

115                 120                 125

Ser Ala Thr Met Glu Gln Ala Val Asn Gln Ala Thr Ala Ser Gly Val
    130                 135                 140

Leu Val Val Ala Ala Ser Gly Asn Ser Gly Ala Gly Asn Val Gly Phe
145                 150                 155                 160

Pro Ala Arg Tyr Ala Asn Ala Met Ala Val Gly Ala Thr Asp Gln Asn
                165                 170                 175

Asn Asn Arg Ala Thr Phe Ser Gln Tyr Gly Ala Gly Leu Asp Ile Val
            180                 185                 190

Ala Pro Gly Val Gly Val Gln Ser Thr Val Pro Gly Asn Gly Tyr Ala
        195                 200                 205

Ser Phe Asn Gly Thr Ser Met Ala Thr Pro His Val Ala Gly Val Ala
    210                 215                 220

Ala Leu Val Lys Gln Lys Asn Pro Ser Trp Ser Asn Val Gln Ile Arg
225                 230                 235                 240

Asn His Leu Lys Asn Thr Ala Thr Asn Leu Gly Asn Thr Thr Gln Phe
                245                 250                 255

Gly Ser Gly Leu Val Asn Ala Glu Ala Ala Thr Arg
            260                 265

<210> 8
<211> 380
<212> PRT
<213> Bacillus alcalophilus

<400> 8
Met Lys Lys Pro Leu Gly Lys Ile Val Ala Ser Thr Ala Leu Leu Ile
  1               5                  10                  15

Ser Val Ala Phe Ser Ser Ser Ile Ala Ser Ala Ala Glu Glu Ala Lys
             20                  25                  30

Glu Lys Tyr Leu Ile Gly Phe Asn Glu Gln Glu Ala Val Ser Glu Phe
         35                  40                  45

Val Glu Gln Val Glu Ala Asn Asp Glu Val Ala Ile Leu Ser Glu Glu
     50                  55                  60

Glu Glu Val Glu Ile Glu Leu Leu His Glu Phe Glu Thr Ile Pro Val
 65                  70                  75                  80

Leu Ser Val Glu Leu Ser Pro Glu Asp Val Asp Ala Leu Glu Leu Asp
                 85                  90                  95

Pro Ala Ile Ser Tyr Ile Glu Glu Asp Ala Glu Val Thr Thr Met Ala
            100                 105                 110

Gln Ser Val Pro Trp Gly Ile Ser Arg Val Gln Ala Pro Ala Ala His

        115                 120                 125

Asn Arg Gly Leu Thr Gly Ser Gly Val Lys Val Ala Val Leu Asp Thr
    130                 135                 140

Gly Ile Ser Thr His Pro Asp Leu Asn Ile Arg Gly Gly Ala Ser Phe
145                 150                 155                 160

Val Pro Gly Glu Pro Ser Thr Gln Asp Gly Asn Gly His Gly Thr His
                165                 170                 175

Val Ala Gly Thr Ile Ala Ala Leu Asn Asn Ser Ile Gly Val Leu Gly
            180                 185                 190

Val Ala Pro Asn Ala Glu Leu Tyr Ala Val Lys Val Leu Gly Ala Ser
        195                 200                 205

Gly Ser Gly Ser Val Ser Ser Ile Ala Gln Gly Leu Glu Trp Ala Gly
    210                 215                 220

Asn Asn Gly Met His Val Ala Asn Leu Ser Leu Gly Ser Pro Ser Pro
225                 230                 235                 240

Ser Ala Thr Leu Glu Gln Ala Val Asn Ser Ala Thr Ser Arg Gly Val
                245                 250                 255

Leu Val Val Ala Ala Ser Gly Asn Ser Gly Ala Gly Ser Ile Ser Tyr
            260                 265                 270

Pro Ala Arg Tyr Ala Asn Ala Met Ala Val Gly Ala Thr Asp Gln Asn
        275                 280                 285

Asn Asn Arg Ala Ser Phe Ser Gln Tyr Gly Ala Gly Leu Asp Ile Val
    290                 295                 300

Ala Pro Gly Val Asn Val Gln Ser Thr Tyr Pro Gly Ser Thr Tyr Ala
305                 310                 315                 320

Ser Leu Asn Gly Thr Ser Met Ala Thr Pro His Val Ala Gly Ala Ala
                325                 330                 335

Ala Leu Val Lys Gln Lys Asn Pro Ser Trp Ser Asn Val Gln Ile Arg
            340                 345                 350

Asn His Leu Lys Asn Thr Ala Thr Ser Leu Gly Ser Thr Asn Leu Tyr
        355                 360                 365

Gly Ser Gly Leu Val Asn Ala Glu Ala Ala Thr Arg
    370                 375                 380

<210> 9
<211> 380
<212> PRT
<213> Bacillus clausii

<400> 9
Met Lys Lys Pro Leu Gly Lys Ile Val Ala Ser Thr Ala Leu Leu Ile

1               5                   10                  15

Ser Val Ala Phe Ser Ser Ser Ile Ala Ser Ala Ala Glu Glu Ala Lys
            20                  25                  30

Glu Lys Tyr Leu Ile Gly Phe Asn Glu Gln Glu Ala Val Ser Glu Phe
        35                  40                  45

Val Glu Gln Val Glu Ala Asn Asp Glu Val Ala Ile Leu Ser Glu Glu
    50                  55                  60

Glu Glu Val Glu Ile Glu Leu Leu His Glu Phe Glu Thr Ile Pro Val
65                  70                  75                  80

Leu Ser Val Glu Leu Ser Pro Glu Asp Val Asp Ala Leu Glu Leu Asp
                85                  90                  95

Pro Ala Ile Ser Tyr Ile Glu Glu Asp Ala Glu Val Thr Thr Met Ala
            100                 105                 110

Gln Ser Val Pro Trp Gly Ile Ser Arg Val Gln Ala Pro Ala Ala His
        115                 120                 125

Asn Arg Gly Leu Thr Gly Ser Gly Val Lys Val Ala Val Leu Asp Thr
    130                 135                 140

Gly Ile Ser Thr His Pro Asp Leu Asn Ile Arg Gly Gly Ala Ser Phe
145                 150                 155                 160

Val Pro Gly Glu Pro Ser Thr Gln Asp Gly Asn Gly His Gly Thr His
                165                 170                 175

Val Ala Gly Thr Ile Ala Ala Leu Asn Asn Ser Ile Gly Val Leu Gly
            180                 185                 190

Val Ala Pro Ser Ala Glu Leu Tyr Ala Val Lys Val Leu Gly Ala Ser
        195                 200                 205

Gly Ser Gly Ser Val Ser Ser Ile Ala Gln Gly Leu Glu Trp Ala Gly
    210                 215                 220

Asn Asn Gly Met His Val Ala Asn Leu Ser Leu Gly Ser Pro Ser Pro
225                 230                 235                 240

Ser Ala Thr Leu Glu Gln Ala Val Asn Ser Ala Thr Ser Arg Gly Val
                245                 250                 255

Leu Val Val Ala Ala Ser Gly Asn Ser Gly Ala Gly Ser Ile Ser Tyr
            260                 265                 270

Pro Ala Arg Tyr Ala Asn Ala Met Ala Val Gly Ala Thr Asp Gln Asn
        275                 280                 285

Asn Asn Arg Ala Ser Phe Ser Gln Tyr Gly Ala Gly Leu Asp Ile Val
    290                 295                 300

Ala Pro Gly Val Asn Val Gln Ser Thr Tyr Pro Gly Ser Thr Tyr Ala
305                 310                 315                 320

Ser Leu Asn Gly Thr Ser Met Ala Thr Pro His Val Ala Gly Ala Ala
                325                 330                 335

Ala Leu Val Lys Gln Lys Asn Pro Ser Trp Ser Asn Val Gln Ile Arg
            340                 345                 350

Asn His Leu Lys Asn Thr Ala Thr Ser Leu Gly Ser Thr Asn Leu Tyr
        355                 360                 365

Gly Ser Gly Leu Val Asn Ala Glu Ala Ala Thr Arg
    370                 375                 380

## Description

### Field of the Invention:

The present invention provides subtilisin variants with improved characteristics, including improved substrate affinity, catalytic activity, catalytic efficiency and stability under washing conditions as well as overall wash performance. The subtilisin variants are therefore useful additives in cleaning compositions.

### Background of the Invention:

Proteases are enzymes that catalyze the hydrolysis of peptide bonds in a protein's backbone. Protease with low substrate specificity are required in order to degrade various protein soils to smaller fragments and, thereby, to support a detergent's action of removing stains like egg, grass, grain etc. from textiles and other materials. Subtilisins are used for this purpose for more than 30 years with large success, mainly as additives in cleaning compositions, e.g. laundry detergents. Consequently, detergent proteases account today for approximately 30% of total worldwide enzyme production. A large number of different subtilisins and subtilisin-like enzymes have been isolated and cloned from several microorganisms including mainly bacillus species (e.g. *Bacillus subtilis, Bacillus lentus, Bacillus licheniformis, Bacillus amyloliquefaciens*), but also from other organisms (e.g. *Tritirachium album, Thermoactinomyces vulgaris).* However, only a few subtilisins have been used commercially as additives for cleaning compositions, mainly due to the fact that the technical conditions under which the enzyme is used are far away from natural conditions. For application in cleaning compositions subtilisins need to be stable and active under the harsh conditions of the automatic washing procedure (medium to high temperature, alkaline pH, oxidizing conditions, surfactants, etc.). Consequently, a large number of studies have been done on the design of subtilisins to fulfill these requirements.

Properties of a protease can be improved by introducing mutations into the amino acid sequence of a precursor, subtilisin at defined positions. Such efforts have lead to better subtilisins as disclosed in several patent applications. For example, US Patent No. 5,346,823 describes subtilisin derivatives which are modified by substitution of methionine, trypthophane, lysine or cysteine with alanine or serine resulting in improved stability under highly oxidizing conditions.

Analogously, a number of subtilisin variants have been obtained that are better suited for technical conditions such as highly alkaline pH, high temperature, high concentrations of detergents, etc. According to US Patent No. 5,246,849 thermally stable serine proteases are created by introducing cysteine at position 206. Subtilisins with enhanced thermal stability were obtained by modifications at the calcium-binding sites at positions 131 and 172 (see US Patent No. 5,260,207). Subtilisin variants showing alterations in characteristics as thermal and alkaline stability were described in US Patent No. 5,972,682. Besides of stability, the utility of an enzyme is further characterized by its catalytical behavior in its proposed technical field. Related parameters such as proteolytic activity, wash performance, were also already subject of several patents. For example, single amino acid substitutions at position 225 and at positions 224 as disclosed in US Patent No. 5,155,033 and in US Patent No. 5,182,204, respectively, resulted in at least 1.5 fold enhanced catalytic activity of the modified subtilisin compared to its precursor subtilisin.

Multi-mutations also can result in increased proteolytic activity as disclosed in EP-A-0451244 describing substitutions at position 123 and 274. US Patent No. 6,287,841 discloses subtilisin mutants derived from PB92 subtilisin or Subtilisin 309 by substitutions at combinations of positions 60, 87, 97, 99, 102, 116, 117, 126, 127, 128, 130, 133, 134, 154, 158, 159, 160, 164, 169, 175, 180, 182, 193, 197, 198, 203, 211 and 216 corresponding to these precursor subtilisins, having improved wash performance or/and improved stability.

Although a lot of progress has been achieved in the field of subtilisin modifications, there is continuing interest in new protease variants with further enhanced characteristics. One major point of common interest is the still enormous amount of protease - be it engineered or not - needed for the technical application in the cleaning process. Taking laundry detergents as an example, the concentration of protease needed for a sufficient removal of protein stains from textiles is between 1 and 5 mg per liter washing liquid. This leads to a worldwide annual consumption of more than 500.000 kg enzyme per year. Furthermore, the high protease concentration corresponds to an increased chance of exposure to the potentially immunogenic protein and, thereby, to an increased probability of allergenic effects.

Therefore, the underlying problem of the present invention is to provide subtilisin variants with improved characteristics, especially subtilisin variants that can be applied technically as additives in cleaning compositions at significantly lower concentrations as compared to known subtilisin variants. Further improved characteristics may include, but are not limited to, higher catalytic rates under application conditions, higher substrate affinity under application conditions, faster removal of protein stains from the particular surface under application conditions, improved stain removing ability at low laundering temperature, better wash performance, improved stability upon storage, improved stability during use, and improved resistance to self-degradation under application conditions. The present invention provides subtilisin variants that are improved for at least one of these characteristics. These subtilisins are useful additives in cleaning compositions such as laundry detergents and other technical or industrial applications.

### Summary of the Invention

The present invention provides
(1) a subtilisin variant having improved characteristics under technical conditions such as a laundry detergent solution, in particular a modified subtilisin comprising at least one mutation in an amino acid sequence of a precursor subtilisin at a position numbered according to the amino acid sequence of the mature subtilisin BPN' shown in SEQ ID NO:1, wherein said at least one mutation is selected from (a) E54D or E54G, (b) Q103R or Q103K, (c) T133K, (d) E156K or E156A, and (e) Y217H, or mutations at the respective positions which are homologous to said mutations (a) to (e), provided that in case of mutations E54D, Q103R E156K or E156A the modified subtilisin has at least one more of said mutations (a) to (e);
(2) a modified subtilisin as defined in (1) above which comprises at least two of the following group of amino acid substitutions: E54D, Q103R, T133K, E156A, Y217H;
(3) a modified subtilisin as defined in (1) to (3) above which is selected form variants 1 to 50 defined hereinbelow;
(4) a DNA coding for the modified subtilisin as defined in (1) to (4) above;
(5) a vector comprising the DNA as defined in (4) above;
(6) a microorganism which comprises the DNA as defined in (4) above and/or the vector as defined in (5) above;
(7) a method for producing the modified subtilisin as defined in (1) to (3) above which comprises culturing a microorganism as defined in (6) above;
(8) a detergent composition comprising a modified subtilisin as defined in (1) to (3) above; and
(9) a method for degrading proteinaceous material which comprises treating the proteinaceous material with a modified subtilisin as defined in (1) to (3) above.

### Short Description of the Figures

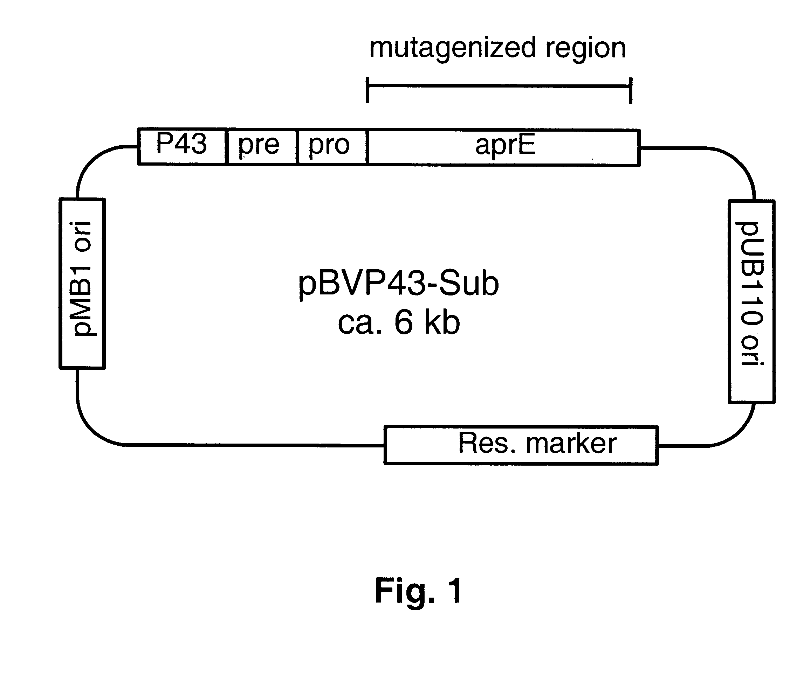

Figure 1 shows a plasmid map of the shuttle vector pBVP43-Sub.

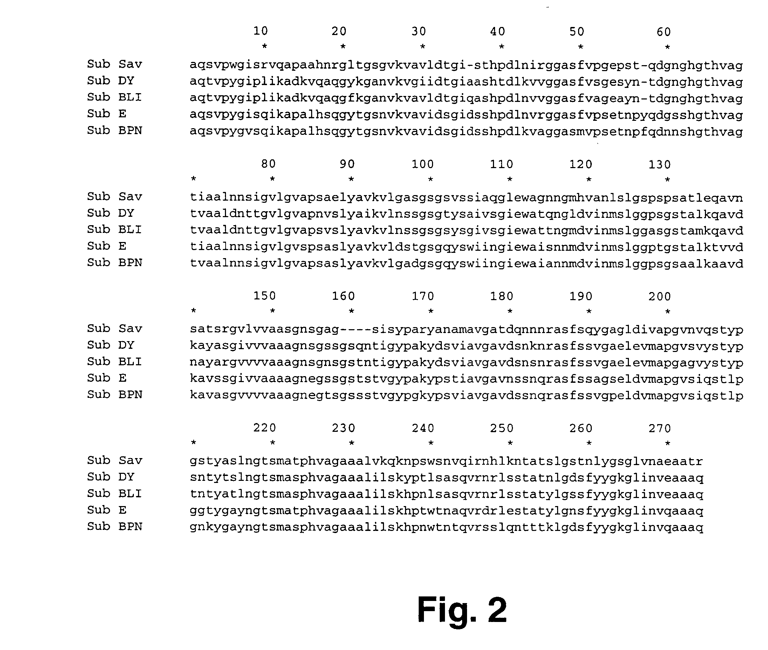

Figure 2 shows an alignment of several subtilisins of the genus Bacillus, wherein Sub DY is subtilisin DY (*Bacillus subtilis*), Sub BLI is subtilisin Carlsberg (*Bacillus licheniformis*), Sub E is subtilisin E (*Bacillus subtilis*), Sub BPN is subtilisin BPN' (*Bacillus amyloliquefaciens*) and Sub Sav is subtilisin Savinase (*Bacillus lentus*).

Figure 3 shows "exemplarily" the improvement of subtilisin variants relativ to the subtilisin E wild type over 4 rounds of directed evolution towards higher activity in laundry detergents.

Figure 4 shows the protein degradation performance of subtilisin variant 22 in comparison to wild type subtilisins. All enzymes were applied at concentrations of 2.5 mg/l.

Figure 5: Determination of the amount of enzyme of variant 50 , 1 , 4 or 22 compared to the subtilisin E wild type needed in order to achieve a certain protein degradation.

Figure 6 shows a Michaelis Menten plot of subtilisin variants 1, 4, 22. All enzymes were applied at concentrations of 2.5 mg/l.

Figure 7 shows a comparison of the protein degradation and solubilization performance between a laundry detergent containing variant 22 and a laundry detergent containing a commercially available subtilisin.

### Detailed Description of the Invention:

The present invention provides subtilisin variants with improved characteristics compared to those of the respective precursor subtilisin enzymes. These improved properties enable a better utility in cleaning compositions.

In the context of this invention "subtilisins" are serine proteases with low substrate specificity and structural homology to subtilisin E from Bacillus subtilis (Biochim. Biophys. Acta 1543 (2000) 203-222). Serine proteases are characterized by the catalytic triade comprising aspartate-histidine-serine, reading from the amino to the carboxy terminus. Well characterized subtilisins derive from bacterial strains, such as subtilisin *E* (*Bacillus subtilis*), subtilisin 309 (*Bacillus lentus*), subtilisin BPN' (*Bacillus amyloliquefaciens*) or subtilisin Carlsberg *(Bacillus licheniformis).* Derived from different species the amino sequences of naturally occuring subtilisins are not entirely homologous, but differ in amino acid substitutions, insertions and deletions. For the purpose of this invention all positions mentioned in the context of subtilisin are equivalent to the numbering of the mature form of the naturally occuring subtilisin BPN' of *Bacillus amyloliquefaciens*.
The amino acids mentioned are abbreviated according to the following list either in one- or in three-letter code.

| **Abbreviations** | | **Amino acid** |
|---|---|---|
| A | Ala | Alanin |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophane |
| Y | Tyr | Tyrosine |

The present invention provides "variants" of subtilisins. A "variant" in accordance with the invention has an amino acid sequence not found in nature, but is instead derived from the amino acid sequence of a "precursor" enzyme by one or more amino acid substitutions (in the present invention also referred to as "mutation"). According to the invention, "precursor" subtilisins can be naturally occuring subtilisins such as subtilisin *E* (*Bacillus subtilis* strain 168; SEQ ID NO:2), subtilisin BPN' (*Bacillus amyloliquefaciens*; SEQ ID NO:1), subtilisin DY (Bacillus subtilis strain DY; SEQ ID NO:3), subtilisin Carlsberg (*Bacillus licheniformis*; SEQ ID NO:4), subtilisin savinase (*Bacillus lentus*; SEQ ID NO:5), or a fragment or derivative thereof, preferably the precursor subtilisin is subtilisin E (SEQ ID NO:2) or a fragment or derivative thereof, or is a recombinant or engineered subtilisin, but also engineered subtilisins having at least 70% homology in its amino acid sequence to a naturally found subtilisin. Furthermore, recombinant subtilisins (being derived from a combination of amino acid sequences of two or more precursor subtilisins) can serve as precursor subtilisins.

By introducing mutations in the amino acid sequence of a precursor subtilisin the characteristics of the resulting subtilisin can be improved. In the context of the present invention, the characteristics of the subtilisin include one, several, or all of the following parameters "Km", "catalytic activity", "catalytic efficiency" and "wash performance" . The Km (or "Michaelis Menten constant") and the catalytic activity are kinetic parameters of an enzyme according to the definitions of Michaelis and Menten (see Fersht, A., Enzyme Structure and Mechanism, W. H. Freeman and Company, New York, 1995). The term Km describes the affinity of the enzymes to its substrate. The "catalytic activity" used herein describes the rate of conversion of the substrate under defined conditions. In the context of this invention, kinetic data are obtained by measuring the hydrolysis of substrates over time. Substrates were fluorescent labeled casein or bovine serum albumin molecules. The degradation was measured over time using confocal fluorescence spectroscopy (see WO9416313). The term "wash performance" used in this invention describes the solubilization and degradation of proteins bound to textiles by the investigated protease applied in a detergent without proteases and compared to the effect of this detergent alone.

In the context of this invention, the term "original amino acid" means the amino acid found in the subtilisin E wild type enzyme at the particular position. It should be noted, that if another subtilisin is to be improved by introducing the amino acid substitutions disclosed in the present invention, the wild type amino acid residue at the particular position might differ from the "original amino acid". Substitutions are usually described as follows: original amino acid; position; substituted amino acid.

The subtilisin variants generated by means of directed evolution as described in the Experimental Section have surprisingly been found to have improved characteristics as industrial enzymes such as detergent proteases.

The subtilisin variants according to embodiment (1) of the present invention comprise one or a combination of the particular mutations (a) to (e), or mutations at the the respective positions which are homologous to said mutations (a) to (e). In particular, the mutation
(a) E54D or E54G means that at position 54, glutamic acid is exchanged by an aspartic acid or a glycine;
(b) Q103R or Q103K means that at position 103, glutamine is exchanged by an arginine or a lysine;
(c) T133K means that at position 133, threonine is exchanged by a lysine;
(d) E156K or E156A means that at position 156, glutamic acid is exchanged by a lysine or an alanine; and
(e) Y217H means that at position 217, tyrosine is exchanged by a histidine.
   "Homologous mutations" according to the present invention are mutations which a person skilled in the art would consider homologous, i.e. equivalent. Thus if the mutated (the newly introduced) amino acid is e.g. a lipophilic amino acid, such as Leu, then other lipophilic amino acids such as Ile are also applicable .

In a preferred embodiment of the invention the subtilisin comprises at least two of the mutations (a) to (e) which includes the following substitution combinations with the numbering of the substitued amio acid resides equivalent to the numbering of Bacillus amyloliquefaciens subtilisin (subtilisin BPN').

E54D/Q103R, E54G/Q103R, E54D/Q103K, E54G/Q103K, E54D/T133K, E54G/T133K, E54D/E156K, E54G/E156K, E54D/E156A, E54G/E156A, E54D/Y217H, E54G/Y217H, Q103R/T133K, Q103R/E156K, Q103R/E156A, Q103R/Y217H, Q103K/T133K, Q103K/E156K, Q103K/E156A, Q103K/Y217H, T133K/E156K, T133K/E156A, T133K/Y217H, E156K/Y217H, E156A/Y217H, E54D/Q103R/T133K, E54G/Q103R/T133K, E54D/Q103R/E156K, E54G/Q103R/E156K, E54D/Q103R/E156A, E54G/Q103R/E156A, E54D/Q103R/Y217H, E54G/Q103R/Y217H, E54D/Q103K/T133K, E54G/Q103K/T133K, E54D/Q103K/E156K, E54G/Q103K/E156K, E54D/Q103K/E156A, E54G/Q103K/E156A, E54D/Q103K/Y217H, E54G/Q103K/Y217H, E54D/T133K/E156K, E54G/T133K/E156K, E54D/T133K/E156A, E54G/T133K/E156A, E54D/T133K/Y217H, E54G/T133K/Y217H, E54D/E156K/Y217H, E54G/E156K/Y217H, E54D/E156A/Y217H, E54G/E156A/Y217H, Q103R/T133K/E156K, Q103R/T133K/E156A, Q103R/T133K/Y217H, Q103R/E156K/Y217H, Q103R/E156A/Y217H, Q103K/T133K/E156K, Q103K/T133K/E156A, Q103K/T133K/Y217H, Q103K/E156K/Y217H, Q103K/E156A/Y217H, T133K/E156K/Y217H, T133K/E156A/Y217H, E54D/Q103R/T133K/E156K, E54G/Q103R/T133K/E156K, E54D/Q103R/T133K/E156A, E54G/Q103R/T133K/E156A, E54D/Q103R/T133K/Y217H, E54G/Q103R/T133K/Y217H, E54D/Q103R/E156K/Y217H, E54G/Q103R/E156K/Y217H, E54D/Q103R/E156A/Y217H, E54G/Q103R/E156A/Y217H, E54D/Q103K/T133K/E156K, E54G/Q103K/T133K/E156K, E54D/Q103K/T133K/E156A, E54G/Q103K/T133K/E156A, E54D/Q103K/T133K/Y217H, E54G/Q103K/T133K/Y217H, E54D/Q103K/E156K/Y217H, E54G/Q103K/E156K/Y217H, E54D/Q103K/E156A/Y217H,E54G/Q103K/E156A/Y217H, E54D/T133K/E156K/Y217H, E54G/T133K/E156K/Y217H, E54D/T133K/E156A/Y217H, E54G/T133K/E156A/Y217H, Q103R/T133K/E156K/Y217H, Q103R/T133K/E156A/Y217H, Q103K/T133K/E156K/Y217H,Q103K/T133K/E156A/Y217H, E54D/Q103R/T133K/E156K/Y217H, E54D/Q103R/T133K/E156A/Y217H, E54G/Q103R/T133K/E156K/Y217H, E54G/Q103R/T133K/E156A/Y217H, E54D/Q103K/T133K/E156K/Y217H, E54D/Q103K/T133K/E156A/Y217H, E54G/Q103K/T133K/E156K/Y217H, E54G/Q103K/T133K/E156A/Y217H.

More preferably the subtilisin of the invention comprises at least three, even more preferably at least four and most preferably at least five of said mutations (a) to (c).

According to the embodiment (2) of the invention, the improved subtilisin variant comprises a combination of at least any two of the following group of amino acid substitutions: E54D, Q103R, T133K, E156A, Y217H.

The subtilisin variants according to embodiments (1) and (2) of the present invention may comprise additional amino acid substitution including substitutions at the following positions: 4, 5, 6, 7, 9, 14, 15, 17, 18, 19, 20, 24, 25, 31, 38, 43, 45, 49, 50, 51, 54 modified differently as compared to mutation (a), 56, 59, 61, 63, 78, 79, 89, 90, 101, 103 modified differently as compared to mutation (b), 104, 111, 118, 130, 133 modified differently as compared to mutation (c), 136, 138, 145, 149, 150, 156 modified differently as compared to mutation (d), 157, 161, 164, 167, 173, 181, 183, 184, 185, 191, 197, 199, 204, 206, 209, 217 modified differently as compared to mutation (e), 218, 224, 228, 234, 235, 242, 243, 245, 247, 248, 249, 251, 252, 253, 259, 263, 265, 271, 267, 269, 271, 272, 273 and 275.

Particularly preferred is that the additional mutation is selected from the following group of amino acid substitutions:
V4A, P5L, Y6N, Y6H, G7S, S9P, S9A, P14T, A15G, A15V, H17L, S18T, Q19H, G20D, S24T, N25D, I31L, S38T, N43S, N43I, N43T, R45S, S49G, F50I, V51I, N56S, Q59L, G61S, S63P, S78P, I79F, S89P, L90I, Y104N, I111V, I111N, N118Y,T130I, T130A, K136N, V138A, S145G, V149I, A150V, E156G, G157D, S161N, T164S, Y167F, N181S, S183N, N184Y, Q185H, S191G, D197G, M199V, S204T, Q206H, Q206L, L209H, N218I, N218T, T224A, A228T, I234N, L235I, L235P, T242A, N2435, N243H, N243T, Q245L, D248V, R247H, R2495, S252G, T2535, N259H, Y263C, Y263N, K265N, L267F, N269H, N269I, N269D, Q271H, Q271L, A272P, Q275L.

In particularly preferred embodiment (3) of the invention, the improved subtilisin is one of the following variants comprising particular combinations of amino acid substitutions (with the numbering of the substituted amino acid resides equivalent to the numbering of subtilisin BPN'):
Variant 1:Y6N/I31L/S38T/F50I/Q103R,
variant 2: P14T/Q103R/T130I/Q185H/K265N,
variant 3: V51I/Q103R/E156A,
variant 4: Q103R/V138A/A150V/E156A/Y217H/Q271H,
variant 5: S9P/R45S/Q103R/T130A/E156G/S183N/Y217H,
variant 6: G61S/S78P/Q103R/E156A/
variant 7: E54D/I79F/Q103R/E156G/Y217H/N243S,
variant 8: S78P/ L90I/Q103R/E156A,
variant 9: Q103K/T130A/E156G/L209H,
variant 10:Q103K/E156A/T242A/N259H,
variant 11: N25D/Q103K/E156A,
variant 12: P14T/A15G/E54D/Q103K/T130A/E156G/S252G/Q271L,
variant 13: Q103K/ T133K/N181S/S252G/Q271L,
varinat 14: N43I/G61S/S78P/Q103K/T130A/E156G/5204T
variant 15: N43S/E54D/Q103R/S145G/E156K/G157D/M199V/Y217H/D248V,
variant 16: S18T/N43S/Q103R/V138A/A150V/E156A/Y217H/Q271L,
variant 17: P14T/A15V/H17L/Q103R/T130A/E156A,
variant 18: Q103R/V138A/A150V/E156A/Y217H/Q271H,
variant 19: Q103R/N181S/S204T/Y217H/T224A/A228T,
variant 20: V4A/E54D/G61S/Q103R/E156A/S183N/Y217H,
variant 21: S9P/E54G/Q103R/I111V/E156A/Y217H,
variant 22: E54D/Q103R/T133K/E156A/Y217H,
variant 23: S24T/G61S/Q103R/E156A/S183N/Y217H/T253S,
variant 24: G20D/N43T/E54D/Q103R/T130A/E156A/S183N/Y217H/L235I,
variant 25: V4A/E54D/G61S/Q103R/S145G/E156A/S183N/Y217H,
variant 26: Q103R/V138A/A150V/E156A/Y217H/D248V/R249S,
variant 27: P14T/A15V/I31L/N43S/E54D/Q103R/E156A,
variant 28: Q19H/I31L/E54D/Q59L/S63P/Q103R/Y104N/T130A/E156G/S161N/S183N/N243H/L267F/N269H,
variant 29: E54D/Q103R/I111N/N118Y/T130A/E156A/Q185H/1234N/L235P/Q245L/N259H,
variant 30: E54D/Q103R/I111N/N118Y/T130A/E156A/Q185H,
variant 31: E54D/T130A/V149I/E156A/S183N/D197G/Q206H/N218I/N269I,
variant 32: E54D/Q103R/T133K/K136N/E156A/N269D,
variant 33: Y6N/P14T/A15V/I31L/N43S/E54D/Q103R/E156A,
variant 34: P14T/A15V/E54D/Q103R/E156A/Y167F/Y217H/N218T,
variant 35: P14T/A15V/I31LyE54D/Q103R/N118Y/E156A/S191G/N243T/Y263C/N269H,
variant 36: G7S/S9A/Q103R/T133K/E156A/Y217H/T224A/Y263N/A272P,
variant 37: E54D/Q103R/T133K/K136N/E156A/Y217H,
variant 38: S9P/Q103R/T133K/E156A/T164S/S183N/Y217H,
variant 39: E54D/Q103R/T133K/E156A/N184Y/Q206L/Y217H/K265N,
variant 40: P5L/Y6H/S49G/Q103R/T133K/E156A/Y217H,
variant 41: E54G/Q103R/I111V/V138A/A150V/E156A/Y217H/L235I,
variant 42:S89P/Q103R/E156A/Q271H,
variant 43: I31L/N43S/N56S/Q103R/V138A/A150V/E156A/Y217H/Q271H,
variant 44: P14T/A15V/E54D/Q103R/E156A/Y217H/Q271H,
variant 45: E54D/Q103R/T133K/E156A,
variant 46: E54D/Q103R/T133K,
variant 47: V4A/E54D/G61S/Q103R/S183N,
variant 48: S9P/E54D/Q103R/I111V/S183N/D248V/R249S,
variant 49: N43S/E54D/Q103R/S145G/E156K/G157D/M199V/Y217H/D248V
variant 50: S145G/E156K/R247H

The substitutions in the amino acid sequence of a precursor subtilisin result in subtilisin variants that surprisingly show enhanced characteristics compared to the precursor subtilisin.The subtilisin variants have distinguishing properties, such as improved catalytic actitvity as compared to the precursor subtilisins. The subtilisin variants of interest in this invention have a lower Km and a higher catalytic activity in comparision to the precursorsubtilisin. Furthermore, the subtilisin variants of interest in this invention surprisingly show an improved wash perfomance in laundry detergent. The improvement of wash performance is connected with a better result in proteinaceous stain removal. or otherwise stated less of the subtilisin variant has to be added to obtain the same result as with the precursor subtilisin. Furthermore, these variants show a better stability in presence of typical ingredients of cleaning composition as oxidizing agents.

Moreover, the invention comprises also the use of the subtilisin variants as defined herein before as additiv in a cleaning composition. This cleaning composition may be used as laundry detergent or a dishwasher detergent.

The invention will now be described in greater detail in the following examples which are ,however, not to be construed to limit the invention.

### Examples:

### Example I: Cloning of the subtilisin E gene and extracelluar expression by B. subtilis

The *aprE* gene coding for subtilisin E was amplified by PCR from the genome of *Bacillus subtilis* strain 168 (DSM #402). Amplification was performed for 20 cycles using a 5:1 mixture of *Taq* and *Pfu* polymerases while *B. subtilis* cells served as templates. Primers simultaneously introducing terminal sites for unique cutting restriction endonucleases were chosen in a way that guaranteed cloning of the full-length CDS into pBV1. pBV1 is an E.coli/B.subtilis shuttle vector that was constructed by introducing a pMB1 origin of replication from pUC19 into pUB110 before deleting its mob gene. Subsequent to cloning of the subtilisin gene into this vector, a synthetic oligomer encoding the P43 promoter was introduced upstream of the gene resulting in pBVP43-Sub. To facilitate mutating the DNA sequence encoding the mature subtilisin without affecting the N-terminal signal sequence, two unique restriction sites were introduced at sites corresponding to the N terminus and the C terminus of the mature subtilisin. The vector construct is shown in Figure
1. Identity of the intermediates as well as the final product of the cloning procedure was confirmed by means of DNA cycle sequencing using the didesoxynucleotide method. Functionality of the construct was confirmed by plating transformants of pBVP43-Sub in an aprE deficient B. subtilis strain (Harwood and Cutting (1990) Molecular Biological Methods for Bacillus, J. Wiley and Sons, New York) on LB agar containing 1% skim milk and the appropriate antibiotics. Subtilisin activity resulted in cleared halos around the Bacillus subtilis colonies. Expression of subtilisin E or a subtilisin variant was done by inoculating complex media containing NaCl, trypton and yeast extract with a *aprE* deficient B. subtilis clone transformed with a pBVP43-Sub plasmid encoding the particular variant, and incubation in Erlenmeyer flasks for 24 to 48 h at 30°C with continuous shaking. After seperation of the cells by centrifugation, either the supernatant was used directly for enzyme characterization, or the enzyme was further purified by salt precipitation, ion exchange chromatography, dialysis and subsequent lyophilization. Concentration of the enzyme in solution was determined by standard protein quantification methods such as the Bradford test. Purity and integrity of the final enzyme was analyzed by SDS polyacrylamide gel electrophoresis.

### Example II: Generation of improved subtilisin variants by means of directed evolution

Subtilisin variants with improved characteristics were generated by means of directed evolution essentially following the protocol provided in WO9218645 in combination with screening methods as provided in WO9416313 or WO9934195.

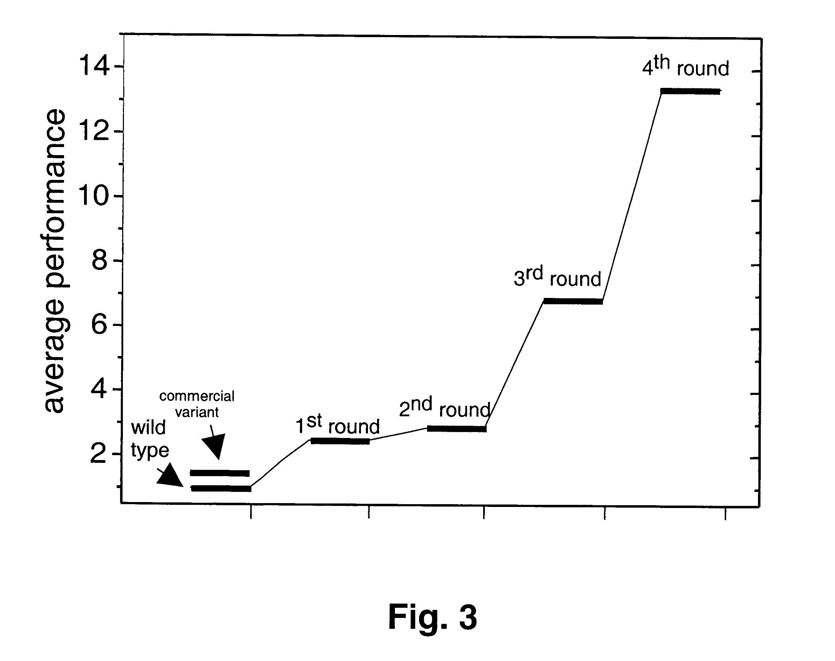

According to these methods, the gene coding for subtilisin E was mutagenized by replication using a DNA polymerase with an error rate in the region of the error threshold. Typically, a modified PCR was used for this purpose. After cloning the DNA product into pBVP43, the library was transformed into an aprE deficient B. subtilis strain. The library was then distributed into the compartments of a suitable sample carrier. Subtilisin variants produced by B. subtilis cells contained in the particular compartments were then examined under conditions relevant to the final application by ultra high throughput screening with confocal fluorescence spectroscopy (see WO9416313 or W09934195). Genes coding for subtilisin variants with improved characteristics under the screening conditions were subsequently selected and isolated by means of PCR amplification, recloned and re-mutagenized. This cycle of mutagenesis, screening and selection was repeated several times. Optionally, the genes coding for selected variants were analyzed by sequencing. As a further option, mutations occuring in different variants selected in a screening round were combined by means of standard restriction and ligation methods or by means of homologous recombination methods, e.g. as provided in WO0134835. An example of the continuous improvement of subtilisin characteristics by directed evolution over 4 rounds is shown in Figure 3.

### Example III : Protein degradation performance of improved subtilisin variants in detergent

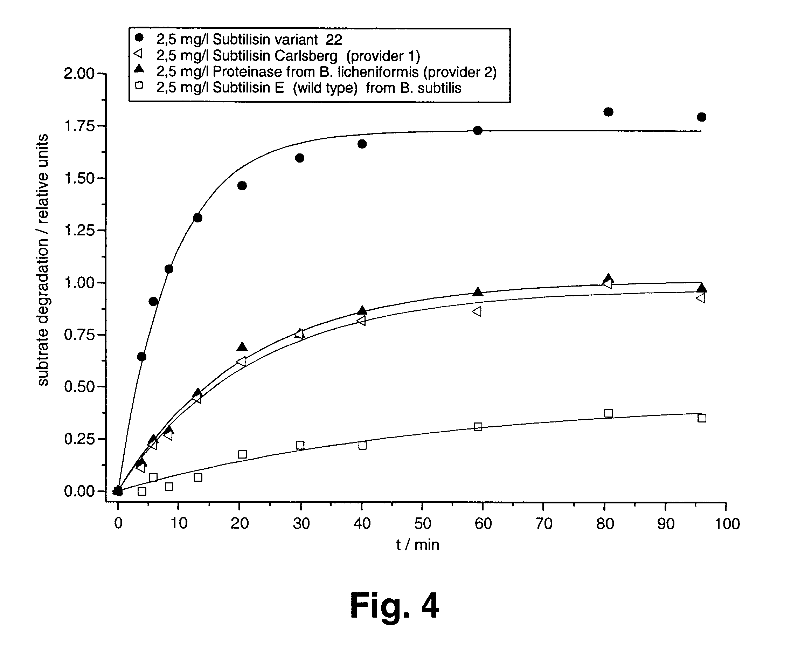

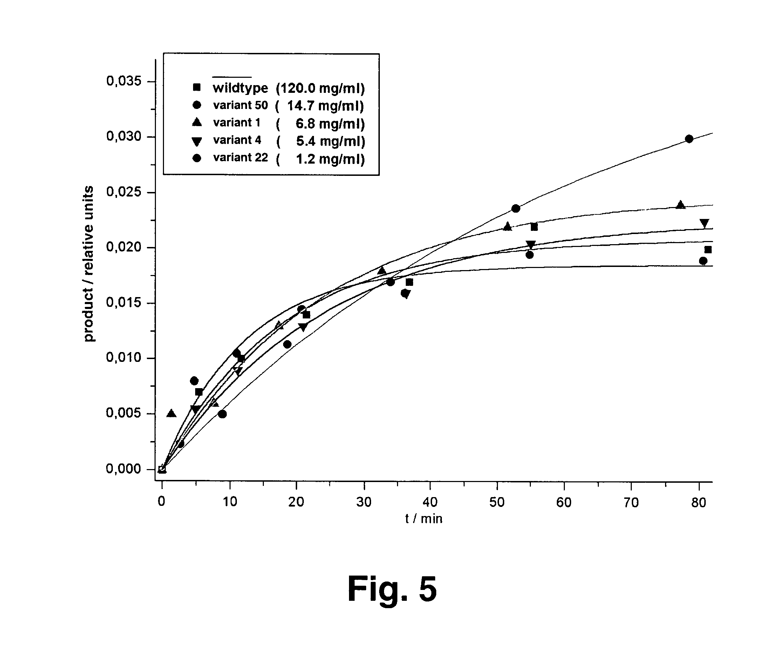

The improvement of subtilisin variants was analyzed by measuring the rate of hydrolysis of protein substrates. Bovine serum albumin (BSA, obtained from Sigma) as an exemplary globular protein was labeled with the fluorescent dye TIRTC (obtained from Sigma) resulting in a labeling degree of approximately one mol dye per mol protein. The degradation of the labelled protein was measured in solution by single-molecule anisotropy, fluorescence correlation spectroscopy and fluorescence intensity measurements using a confocal fluorescence spectroscopy set-up (see WO9416313). For further evaluation, FITC-labelled casein (casein obtained from Sigma, FITC obtained from Fluka) was used as a substrate. For analyzing the characteristics of enzyme variants under washing conditions, tests were carried out in a freshly prepared typical laundry detergent solution. In Figure 4 the degradation of an improved subtilisin variant is shown in comparison to the wild type as well as another commercially applied subtilisin, Subtilisin Carlsberg from B. licheniformisobtained from two sources, at typical concentrations. In Figure 5 another set of four variants is compared in terms of the amount of enzyme needed to catalyze protein degradation with the same rate. As can be easily seen, the best variant out of this set has the same catalytic activity at 1/100 concentration compared to the wild type. Table I gives an overview of several variants and their degradation performance is measured as protein degradation in detergent after a periode of 30 minutes normalized to the wild type.

**Table I**

| subtilisin | relative protein degradation performance |
|---|---|
| wild type | 1,00 |
| variant 1 | 3,91 |
| variant 2 | 1,90 |
| variant 3 | 4,34 |
| variant 4 | 6,85 |
| variant 5 | 2,18 |
| variant 7 | 5,29 |
| variant 8 | 2,26 |
| variant 9 | 2,06 |
| variant 10 | 4,56 |
| variant 11 | 4,26 |
| variant 12 | 2,81 |
| variant 13 | 1,84 |
| variant 15 | 4,30 |
| variant 16 | 6,57 |
| variant 17 | 5,85 |
| variant 18 | 6,60 |
| variant 19 | 6,35 |
| variant 20 | 7,45 |
| variant 21 | 8,50 |
| variant 22 | 8,37 |
| variant 23 | 8,04 |
| variant 24 | 7,63 |
| variant 25 | 7,74 |
| variant 26 | 7,79 |
| variant 27 | 6,16 |
| variant 28 | 5,98 |
| variant 29 | 5,75 |
| variant 30 | 6,73 |
| variant 31 | 5,95 |
| variant 32 | 6,77 |
| variant 33 | 6,67 |
| variant 34 | 7,24 |
| variant 35 | 4,12 |
| variant 36 | 6,89 |
| variant 37 | 7,45 |
| variant 38 | 6,13 |
| variant 39 | 7,22 |
| variant 40 | 5,91 |
| variant 41 | 5,73 |
| variant 42 | 3,77 |
| variant 43 | 4,99 |
| variant 44 | 3,36 |

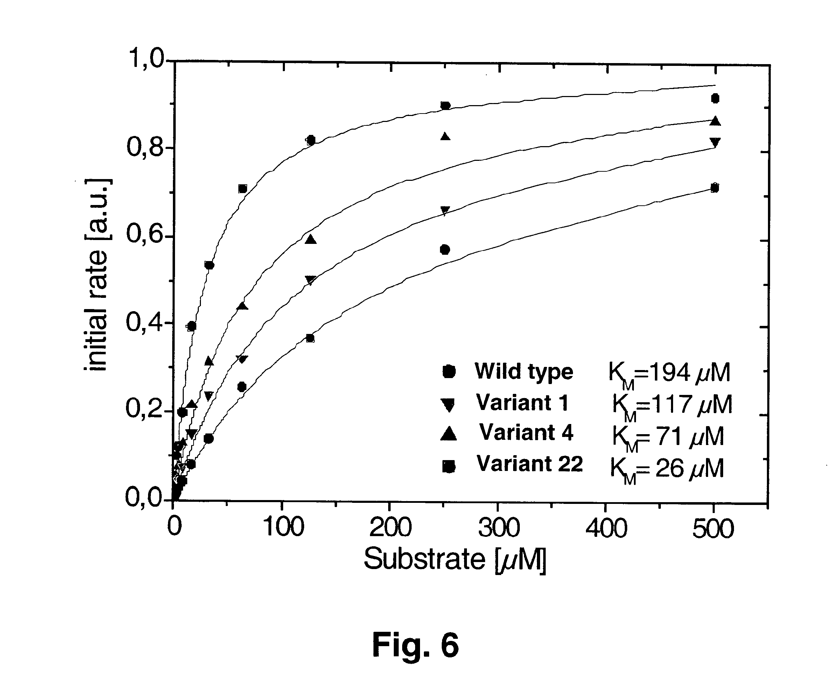

Figure 6 and Table 1 compare the Km of some of these variants with the Km of the wild type enzyme.

**Table II**

| Subtilisin | Km [µM] |
|---|---|
| Subtilisin E (wild type) | 194 |
| Variant 1 | 117 |
| Variant 4 | 71 |
| Variant 22 | 26 |

### Example IV: Effect of improved variants on protein solubilization and subsequent degradation from textiles

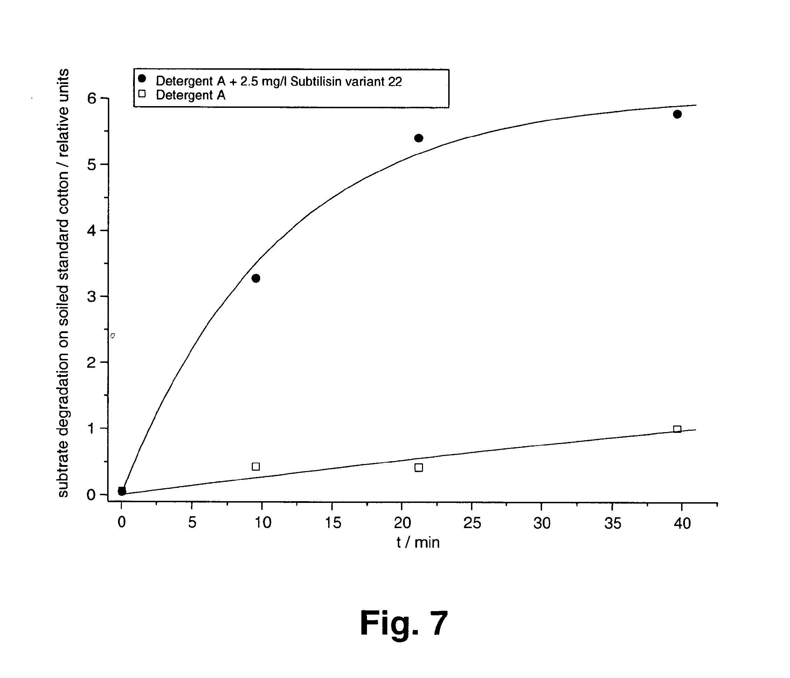

In order to analyze the degradation of proteins bound to textile surfaces and, thereby, the efficieny of a particular subtilisin variant in removing protein stains from soiled textiles, a solid phase assay was used. Standard cotton (Nr. 10A, wfk Testgewebe GmbH, Christenfeld 10, D41379 Brüggen-Bracht, Germany) was impregnated with 20% hen egg solution containing TIRTC labelled BSA. Then the cotton was cut into pieces, dried, washed three times with laundry detergent containing no protease, dried again and then incubated with laundry detergent containing the particular subtilisin variant, or commercially available subtilisin variant, both at 2.5 mg/l, or no protease as control. The release of proteins from the textile and the further degradation in solution was then measured by single-molecule anisotropy, fluorescence correlation spectroscopy and fluorescence intensity measurements using a confocal fluorescence spectroscopy set-up (see WO9416313). Figure 7 compares the rate of release of labelled protein fragments catalyzed by a particular variant with the rate of a comercially available protease.

## Claims

1. A modified subtilisin comprising at least one mutation in an amino acid sequence of a precursor subtilisin at a position numbered according to the amino acid sequence of the mature subtilisin BPN' shown in SEQ ID NO:1, wherein said at least one mutation is selected from (a) E54D or E54G, (b) Q103R or Q103K, (c) T133K, (d) E156K or E156A, and (e) Y217H, or mutations at the respective positions which are homologous to said mutations (a) to (e), provided that in case of mutations E54D, Q103R, E156K or E156A the modified subtilisin has at least one more of said mutations (a) to (e).

2. The modified subtilisin of claim 1 which comprises at least two (2) preferably at least three (3), more preferably at least four (4) and most preferably at least five (5) of said mutations (a) to (e).

3. The modified subtilisin of claim 1 or 2 which comprises at least two (2) preferably at least three (3), more preferably at least four (4) and most preferably at least five (5) of the following group of amino acid substitutions: E54D, Q103R, T133K, E156A, Y217H.

4. The modified subtilisin of any one of claims 1 to 3 which further comprises mutation at the following amino acid residue positions of the precursor subtilisin: 4, 5, 6, 7, 9, 14, 15, 17, 18, 19, 20, 24, 25, 31, 38, 43, 45, 49, 50, 51, 54 modified differently as compared to mutation (a), 56, 59, 61, 63, 78, 79, 89, 90, 101, 103 modified differently as compared to mutation (b), 104, 111, 118, 130, 133 modified differently as compared to mutation (c), 136, 138, 145, 149, 150, 156 modified differently as compared to mutation (d), 157, 161, 164, 167, 173, 181, 183, 184, 185, 191, 197, 199, 204, 206, 209, 217 modified differently as compared to mutation (e), 218, 224, 228, 234, 235, 242, 243, 245, 247, 248, 249, 251, 252, 253, 259, 263, 265, 271, 267, 269, 271, 272, 273 and 275; preferably said modified subtilisin further comprises one or more of the following mutations: V4A, P5L, Y6N, Y6H, G7S, S9P, S9A, P14T, A15G, A15V, H17L, S18T, Q19H, G20D, S24T, N25D, I31L, S38T, N43S, N43I, N43T, R45S, S49G, F50I, V51I, N56S, Q59L, G61S, S63P, S78P, I79F, S89P, L90I, Y104N, I111V, I111N, N118Y, T130I, T130A, K136N, V138A, S145G, V149I, A150V, E156G, G157D, S161N, T164S, Y167F, N181S, S183N, N184Y, Q185H, S191G, D197G, M199V, S204T, Q206H, Q206L, L209H, N218I, N218T, T224A, A228T, I234N, L235I, L235P, T242A, N243S, N243H, N243T, Q245L, R247H, D248V, R249S, S252G, T253S, N259H, Y263C, Y263N, K265N, L267F, N269H, N269I, N269D, Q271H, Q271L, A272P and Q275L.

5. The modified subtilisin according to any one of claims 1 to 4 which has one of the following combinations of mutations:
V51I/Q103R/E156A, Q103R/V138A/A150V/E156A/Y217H/Q271H, S9P/R45S/Q103R/T130A/E156G/S183N/Y217H,G61S/S78P/Q103R/E156A/E54D/I79F/Q103R/E156G/Y217H/N243S, S78P/L90I/Q103R/E156A, Q103K/T130A/E156G/L209H, Q103K/E156A/T242A/N259H, N25D/Q103K/E156A, P14T/A15G/E54D/Q103K/T130A/E156G/S252G/Q271L, Q103K/ T133K/N181S/S252G/Q271L, N43I/G61S/S78P/Q103K/T130A/E156G/S204T N43S/E54D/Q103R/S145G/E156K/G157D/M199V/Y217H/D248V, S18T/N43S/Q103R/V138A/A150V/E156A/Y217H/Q271L, P14T/A15V/H17L/Q103R/T130A/E156A,Q103R/V138A/A150V/E156A/Y217H, Q103R/N181S/S204T/Y217H/T224A/A228T, V4A/E54D/G61S/Q103R/E156A/S183N/Y217H, S9P/E54G/Q103R/I111V/E156A/Y217H, E54D/Q103R/T133K/E156A/Y217H, S24T/G61S/Q103R/E156A/S183N/Y217H/T253S, G20D/N43T/E54D/Q103R/T130A/E156A/S183N/Y217H/L235I, V4A/E54D/G61S/Q103R/S145G/E156A/S183N/Y217H, Q103R/V138A/A150V/E156A/Y217H/D248V/R249S, P14T/A15V/I31L/N43S/E54D/Q103R/E156A, Q19H/I31L/E54D/Q59L/S63P/Q103R/Y104N/T130A/E156G/S161N/S183N/N243H/L267F/N269H, E54D/Q103R/I111N/N118Y/T130A/E156A/Q185H/I234N/L235P/Q245L/N259H, E54D/Q103R/I111N/N118Y/T130A/E156A/Q185H, E54D/T130A/V149I/E156A/S183N/D197G/Q206H/N218I/N269I, E54D/Q103R/T133K/K136N/E156A/N269D, Y6N/P14T/A15V/I31L/N43S/E54D/Q103R/E156A, P14T/A15V/E54D/Q103R/E156A/Y167F/Y217H/N218T, P14T/A15V/I31L/E54D/Q103R/N118Y/E156A/S191G/N243T/Y263C/N269H, G7S/S9A/Q103R/T133K/E156A/Y217H/T224A/Y263N/A272P, E54D/Q103R/T133K/K136N/E156A/Y217H, S9P/Q103R/T133K/E156A/T164S/S183N/Y217H, E54D/Q103R/T133K/E156A/N184Y/Q206L/Y217H/K265N, P5L/Y6H/S49G/Q103R/T133K/E156A/Y217H, E54G/Q103R/I111V/V138A/A150V/E156A/Y217H/L235I, S89P/Q103R/E156A/Q271H, I31L/N43S/N56S/Q103R/V138A/A150V/E156A/Y217H/Q271H, P14T/A15V/E54D/Q103R/E156A/Y217H/Q271H, E54D/Q103R/T133K/E156A, E54D/Q103R/T133K, V4A/E54D/G61S/Q103R/S183N, S9P/E54D/Q103R/I111V/S13N/D248V/R249S and N43S/E54D/Q103R/S145G/E156K/G157D/M199V/Y217H/D248V.

6. A modified subtilisin comprising the following combination of mutations in an amino acid sequence of a precursor subtilisin at a position numbered according to the amino acid sequence of the mature subtilisin BPN' shown in SEQ ID NO:1: Y6N/I31L/S38T/F50I/Q103R, P14T/Q103R/T130I/Q185H/K265N, and S145/E156/R247H.

7. The modified subtilisin according to any opne of claims 1 to 6, wherein the precursor subtilisin is subtilisin E (*Bacillus subtilis* strain 168; SEQ ID NO:2), subtilisin BPN' (*Bacillus amyloliquefaciens*; SEQ ID NO:1), subtilisin DY (Bacillus subtilis strain DY; SEQ ID NO:3), subtilisin Carlsberg (*Bacillus licheniformis;* SEQ ID NO:4), subtilisin savinase (*Bacillus lentus;* SEQ ID NO:5), or a fragment or derivative thereof, preferably the precursor subtilisin is subtilisin E (SEQ ID NO:2) or a fragment or derivative thereof, or is a recombinant or engineered subtilisin.

8. A DNA coding for the modified subtilisin of claims 1 to 7.

9. A vector comprising the DNA of claim 8.

10. A microorganism which comprises the DNA of claim 8 and/or the vector of claim 8.

11. A method for producing the modified subtilisin of claims 1 to 7 which comprises culturing the microorganism of claim 10.

12. A detergent composition comprising a modified subtilisin of claims 1 to 7.

13. A method for degrading protinaceous material which comprises treating the proteinaceous material with a modified subtilisin of claims 1 to 7.
